(19) Europäisches Patentamt
European Patent Office
Office européen des brevets

(11) EP 0 786 450 B1

(12) **EUROPÄISCHE PATENTSCHRIFT**

(45) Veröffentlichungstag und Bekanntmachung des Hinweises auf die Patenterteilung:
**05.04.2000 Patentblatt 2000/14**

(51) Int Cl.[7]: **C07C 213/08**, C07C 213/10

(21) Anmeldenummer: **96120727.1**

(22) Anmeldetag: **21.12.1996**

(54) **Verfahren zur Herstellung der Enantiomeren von O-Demethyltramadol**

Process for the preparation of O-demethyltramadol enantiomers

Procédé pour la préparation des enantiomères du O-deméthyltramadol

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **19.01.1996 DE 19601744**

(43) Veröffentlichungstag der Anmeldung:
**30.07.1997 Patentblatt 1997/31**

(73) Patentinhaber: **Grünenthal GmbH**
**52078 Aachen (DE)**

(72) Erfinder:
- **Buschmann, Helmut, Dr.**
  **52066 Aachen (DE)**
- **Jansen, Peter**
  **52249 Eschweiler (DE)**
- **Winter, Werner, Prof.-Dr.**
  **52078 Aachen (DE)**
- **Strassburger, Wolfgang, Prof.-Dr.**
  **52146 Würselen (DE)**
- **Graudums, Ivars, Dr.**
  **52222 Stolberg (DE)**
- **Friderich, Elmar, Dr.**
  **52223 Stolberg (DE)**

(56) Entgegenhaltungen:
**GB-A- 1 084 006**

- **ARZNEIM.-FORSCH. (1978), 28(1A), 114-21 CODEN: ARZNAD;ISSN: 0004-4172, 1978, XP000644506 FRANKUS, E. ET AL: "Separation of isomers, structural elucidation and pharmacological characterization of 1-(m-methoxyphenyl)-2-(dimethylaminomethyl ) -1- cyclohexanol"**
- **J. JACQUES ET AL.: "Enantiomers, racemates and resolutions" 1991 , KRIEGER PUBLISHING COMPANY , MALABAR, FLORIDA (U.S.A) XP002030514 20422 * Seite 259 - Seite 261 * * Seite 387 - Seite 388 ***
- **SYNTHESIS, Oktober 1975, STUTTGART DE, Seiten 617-630, XP002030544 E. WINTERFELDT: "Applications of diisobutylaluminium hydride (DIBAH) and triisobutylaluminium as reducing agents in organic synthesis"**
- **CHEMICAL ABSTRACTS, vol. 121, no. 19, 7.November 1994 Columbus, Ohio, US; abstract no. 220754, DAYER, PIERRE ET AL: "The pharmacology of tramadol" XP002030545 & DRUGS (1994), 47(SUPPL. 1), 3-7 CODEN: DRUGAY;ISSN: 0012-6667, 1994,**
- **CHEMICAL ABSTRACTS, vol. 119, no. 25, 20.Dezember 1993 Columbus, Ohio, US; abstract no. 262330, SEVCIK, JAN ET AL: "Effects of the central analgesic tramadol and its main metabolite, O-desmethyltramadol, on rat locus coeruleus neurons" XP002030546 & BR. J. PHARMACOL. (1993), 110(1), 169-76 CODEN: BJPCBM;ISSN: 0007-1188, 1993,**
- **ARZNEIMITTEL FORSCHUNG. DRUG RESEARCH, Bd. 28, Nr. 1A, 1.Januar 1978, Seiten 107-113, XP000608150 FLICK K ET AL: "UNTERSUCHUNGEN ZUR CHEMISCHEN STRUKTUR UND ANALGETISCHEN WIRKUNG VON PHENYLSUBSTITUIERTEN AMINOMETHYLCYCLOHEXANOLEN STUDIES ON CHEMICAL STRUCTURE AND ANALGETIC ACTIVITY OF PHENYL SUBSTITUTED AMINOMETHYLCYCLOHEXANOLES"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 786 450 B1

## Beschreibung

Die Erfindung betrifft ein Verfahren zur Herstellung der Enantiomeren von o-Demethyltramadol.

Opioide werden seit vielen Jahren als Analgetika zur Schmerzbehandlung eingesetzt, obwohl sie eine Reihe von Nebenwirkungen, beispielsweise Sucht und Abhängigkeit, Atemdepression, gastro-intestinale Hemmwirkung und Obstipation, hervorrufen. Sie können daher nur unter besonderen Vorsichtsmaßnahmen wie speziellen Verordnungsvorschriften über einen längeren Zeitraum oder in höheren Dosierungen gegeben werden (Goodman, Gilman, The Pharmacological Basis of Therapeutics, Pergamon Press, New York 1990).

Tramadolhydrochlorid - (1RS,2RS)-2-Dimethylaminomethyl1-(3-methoxy-phenyl)-cyclohexanol, Hydrochlorid - nimmt unter den zentralwirksamen Analgetika eine Sonderstellung ein, da dieser Wirkstoff eine starke Schmerzhemmung ohne die für Opioide bekannten Nebenwirkungen hervorruft (J. Pharmacol. Exptl. Ther. 267, 331 (1993)). Tramadol ist ein Racemat und besteht aus gleichen Mengen an (+)- und (-)-Enantiomer. In vivo bildet dieser Wirkstoff den Metaboliten O-Demethyltramadol, der gleichfalls als Enantiomerengemisch vorliegt. Untersuchungen haben ergeben, daß sowohl die beiden Tramadol-Enantiomere als auch die beiden Enantiomere der Tramadolmetabolite an der analgetischen Wirkung beteiligt sind (J. Pharmacol. Exptl. Ther. 260, 275 (1992); Arzneim.-Forschung 38, 877 (1988)).

Die Herstellung von O-Demethyltramadol als Racemat oder in Form der Enantiomeren ist aus EP 534 628 und WO 93/04675 bekannt. Nach diesem Verfahren, das mit einer starken Base wie Natrium- oder Kaliumhydrid in Gegenwart von Thiophenol und Diethylenglycol durchgeführt wird, läßt sich O-Demethyltramadol jedoch nur in unbefriedigenden Ausbeuten erhalten. Die der Erfindung zugrundeliegende Aufgabe bestand daher in der Entwicklung eines Verfahrens, mit dem sich O-Demethyltramadol in hohen Ausbeuten herstellen läßt.

Es wurde gefunden, daß mit dem Einsatz von L-(+)-Weinsäure zur Racematspaltung von Tramadol und anschließender Methyletherspaltung mit Diisobutylaluminiumhydrid (DIBAH) O-Demethyltramadol in enantiomerenreiner Form in hoher Ausbeute hergestellt werden kann.

Erfindungsgegenstand ist dementsprechend ein Verfahren zur Herstellung der Enantiomeren von O-Demethyltramadol, welches dadurch gekennzeichnet ist, daß man ein racemisches Tramadolsalz in die Base überführt, mit L-(+)-Weinsäure das (-)-Tramadol-Enantiomere durch Fällung abtrennt und nach Freisetzung der Base in das (-)-Enantiomere von O-Demethyltramadol mit DIBAH überführt, und aus der Mutterlauge der Weinsäurefällung das (+)-Enantiomere von O-Demethyltramadol durch Freisetzung der Tramadol-Base und Umsetzung mit DIBAH herstellt.

Für das erfindungsgemäße Verfahren eignet sich als Edukt insbesondere racemisches Tramadolhydrochlorid. Dieses wird in einer wäßrigen Lösung unter Zusatz von Alkalihydroxiden, vorzugsweise von Natriumhydroxid, und Extraktion mit einem organischen Lösungsmittel, beispielsweise Dichlormethan und/oder Diethylether, in racemische Tramadolbase überführt. Anschließend wird die erhaltene Base mit L-(+)-Weinsäure vorzugsweise in Gegenwart eines organischen Lösungsmittels, besonders bevorzugt in Gegenwart eines aliphatischen $C_{1-5}$-Alkohols versetzt. Das sich bildende Tartrat des (-)-Tramadol-Enantiomeren wird vorzugsweise durch Kristallisation von dem gebildeten Tartrat des (+)-Tramadol-Enantiomeren abgetrennt und nach Freisetzung der Tramadolbase unter den vorgenannten Bedingungen mit DIBAH in das (-)-Enantiomere von O-Demethyltramadol überführt. Die Methyletherspaltung mit DIBAH wird üblicherweise in einem aromatischen Kohlenwasserstoff, beispielsweise Toluol bei einer Temperatur zwischen 60 und 130° C durchgeführt.

Das in der Mutterlauge lösliche (+)-Tramadol-Enantiomere in Form des Tartratsalzes wird durch Freisetzung der Tramadolbase unter den vorgenannten Bedingungen und anschließender Umsetzung mit DIBAH unter den vorgenannten Bedingungen in das (+)-Enantiomere von O-Demethyltramadol überführt.

Die erhaltenen Enantiomere von O-Demethyltramadol können als Basen oder als Salze, insbesondere als Hydrochloride isoliert werden. Die Hydrochloride sind unter den gleichen Bedingungen wie die Hydrochloride von Tramadol erhältlich.

Vor der Umsetzung mit DIBAH ist es vorteilhaft, die aus dem Tartrat des jeweiligen Tramadol-Enantiomeren freigesetzte Base in ein von Tartrat verschiedenes Tramadolsalz, vorzugsweise in ein Hydrochlorid zu überführen und aus diesem wiederum die Tramadolbase unter den vorgenannten Bedingungen freizusetzen.

Die Umwandlung der Tramadol-Base in das Hydrochlorid kann mit konzentrierter Salzsäure oder gasförmigem Chlorwasserstoff in einem organischen Lösungsmittel, beispielsweise Aceton, Dioxan, Diethylether und/oder Diisopropylether, oder mit Trimethylchlorsilan/Wasser in einem Lösungsmittel, beispielsweise 2-Butanon durchgeführt werden.

Mit dem erfindungsgemäßen Verfahren lassen sich die Enantiomeren von O-Demethyltramadol kostengünstig, umweltfreundlich und in hohen Ausbeuten herstellen. Zur Racematspaltung eines Tramadolsalzes ist nur eine enantiomere Form der Weinsäure, nämlich die kostengünstige L-(+)-Weinsäure erforderlich. Mit L-(+)-Weinsäure lassen sich die Tramadol-Enantiomeren, bezogen auf eingesetztes Racemat in über 85 %iger Ausbeute mit einer Enantiomeren-Reinheit von über 98 % erhalten. Nach Freisetzung der Tramadolbase kann die Mutterlauge in den Racematspaltungsprozeß zurückgeführt werden. Die Methyletherspaltung ergibt in über 95 %iger Ausbeute die Enantiomeren von O-Demethyltramadol.

In EP 534 628 und WO 93/04675 ist die Verwendung von O-Demethyltramadol in Kombination mit Codein, Oxycodon, Hydrocodon oder Acetaminophen zur Behandlung von Schmerzzuständen beschrieben. Es wurde nunmehr gefunden, daß bereits O-Demethyltramadol alleine oder in Kombination mit Tramadol eine hohe analgetische Wirkung besitzt.

Die erfindungsgemäßen Analgetika enthalten neben der Base und/oder mindestens einem Salz von O-Demethyltramadol alleine oder in Kombination mit Tramadol-Base und/oder mindestens einem Tramadolsalz Trägermaterialien, Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, intravenös, buccal, intraperitoneal, intradermal, intramuskulär, intranasal oder örtlich, zum Beispiel an der Haut, den Schleimhäuten oder den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Die erfindungsgemäß zu verwendenden Verbindungen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäß zu verwendenden Verbindungen verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 5 bis 500 mg/kg wenigstens einer der oben genannten Verbindungen appliziert.

## Beispiele

### Beispiel 1

(-)-(1S,2S)-2-Dimethylaminomethyl-1-(3-methoxy-phenyl)-cyclohexanol, Hydrochlorid (-1)

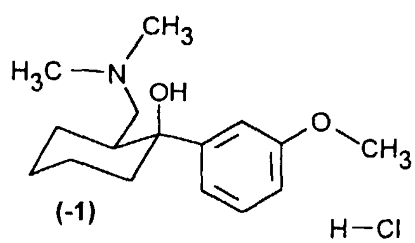

**(-1)**

1. Stufe: Freisetzung der racemischen Base

3 kg (10 mol) (1RS, 2RS)-2-Dimethylaminomethyl-1-(3-methoxy-phenyl)-cyclohexanol, Hydrochlorid (1) wurden in 4800 ml Wasser suspendiert und mit 1,6 kg zerstoßenem Eis versetzt. Unter Rühren wurden 1300 ml 36-38 %ige Natronlauge (technisch) zugetropft. Anschließend wurde mit 7000 ml Dichlormethan und nach Phasentrennung mit weiteren 2000 ml Dichlormethan extrahiert. Die vereinigten organischen Phasen wurden über Natriumsulfat getrocknet. Nach destillativer Entfernung des Lösungsmittels wurden 2630 g (99 % der Theorie) (1RS, 2RS)-2-Dimethylaminomethyl-1-(3-methoxy-phenyl)-cyclohexanol als Sirup erhalten.

2. Stufe: Fällung mit L-(+)-Weinsäure

2630 g (10 mol) der Base aus der ersten Stufe wurden in 2400 ml Ethanol gelöst und mit einer Lösung bestehend aus 1500 g (10 mol) L-(+)-Weinsäure und 11200 ml Ethanol versetzt. Zur Kristallisation wurde zwei Stunden bei Raumtemperatur gerührt und 24 Stunden bei 4° C stehen gelassen. Die ausgefallenen Kristalle wurden abgesaugt und mit 6400 ml Ethanol von 4° C gewaschen. Nach Trocknung des Kristallisates bei Raumtemperatur im Vakuum (60 mbar) wurden 2050 g (49 % bezogen auf die Gesamtmenge an eingesetzter racemischer Base) (1S, 2S)-2-Dimethylaminomethyl-1-(3-methoxy-phenyl)-cyclohexanol-L-(+)-tartrat mit einem Schmelzpunkt von 173-175° C erhalten (Drehwert: $[\alpha]_D^{RT}$ = -12,2° (c = 1,01; Methanol).

3. Stufe: Freisetzen der Base aus dem L-(+)-Weinsäuresalz

2050 g (4,95 mol) (1S, 2S)-2-Dimethylaminomethyl-1-(3-methoxy-phenyl)-cyclohexanol-L-(+)-tartrat aus Stufe 2 wurden in 4000 ml Wasser gelöst und mit 900 g zerstoßenem Eis versetzt. Unter Rühren wurden 1000 ml 36-38 %ige Natronlauge (technisch) zugetropft. Anschließend wurde mit 2500 ml Dichlormethan und nach Phasentrennung mit weiteren 500 ml Dichlormethan extrahiert. Die vereinigten organischen Phasen wurden über Natriumsulfat getrocknet. Nach destillativer Entfernung des Lösungsmittels wurden 1280 g (99 % der Theorie) (1S,

2S)-2-Dimethylaminomethyl-1-(3-methoxy-phenyl)-cyclohexanol als Sirup erhalten.

4. Stufe: Überführung von (1S, 2S)-2-Dimethylamino-methyl-1-(3-methoxy-phenyl)-cyclohexanol in das Hydrochlorid (-1)

1280 g (4,86 mol) der aus Stufe 3 erhaltenen Base wurden in 16 l 2-Butanon gelöst und unter Rühren mit 88 ml (4,9 mol) Wasser sowie 621 ml (532 g; 4,9 mol) Trimethylchlorsilan versetzt. Zur Kristallisation wurde 3 Stunden bei Raumtemperatur gerührt und 24 Stunden bei 4° C stehen gelassen. Der ausgefallene Feststoff wurde abgesaugt, mit 5000 ml 2-Butanon von 4° C gewaschen und bei 90° C im Vakuum (60 mbar) bis zur Gewichtskonstanz getrocknet. Es wurden 1390 g (95 % der Theorie bezogen auf die eingesetzte Base aus Stufe 3 und 92 % bezogen auf den Enantiomerenanteil des eingesetzten Racemates aus Stufe 1) Hydrochlorid (-1) als farblose Kristalle erhalten.

Schmelzpunkt: 172-173° C
Drehwert: $[\alpha]_D^{RT}$ = -29,6° (c = 1,00; Methanol)

5. Stufe: Überführung von Hydrochlorid (-1) in (-)-(1S, 2S)-3-(2-Dimethylaminomethyl-1-hydroxy-cyclohexyl)-phenol, Hydrochlorid

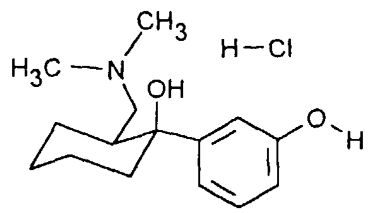

Aus dem Hydrochlorid (-1) wurde mit Dichlormethan/Natriumhydroxid-Lösung unter den in der 1. Stufe angegebenen Bedingungen die Base freigesetzt. Nach dem Trocknen der Lösung wurde Dichlormethan im Vakuum abdestilliert. 208,1 g (0,79 mol) der erhaltenen Base, gelöst in 360 ml Toluol, wurden bei Raumtemperatur zu 1,6 1 20 %iger Diisobutylaluminiumhydrid-Lösung (1,58 mol) in Toluol getropft. Anschließend wurde 11 Stunden unter Rückfluß erhitzt und nach Abkühlung auf Raumtemperatur mit Eis/Kochsalz auf ca. 0° C gekühlt. Dann wurden 450 ml Ethanol so zugetropft, daß die Innentemperatur 15° C nicht überstieg. Nach vollständiger Zugabe wurde 15 Minuten weitergerührt und mit 1000 ml Toluol verdünnt. Unter Eis/Kochsalzkühlung wurden 450 ml eines Ethanol/Wasser-Gemisches (1 : 1) zugetropft und nach vollständiger Zugabe eine Stunde bei Raumtemperatur gerührt. Das ausgefallene Aluminiumhydroxid wurde abgesaugt und mit fünf Volumenteilen Essigsäureethylester bei 60° C zur Nachextraktion verrührt. Nach erneutem Absaugen wurden die vereinigten organischen Phasen über Natriumsulfat getrocknet und am Rotationsverdampfer bei 60° C eingeengt. Es wurden 193 g (98 % der Theorie) Base erhalten, wobei die Base als Feststoff mit einem Schmelzpunkt von 139-142 °C auskristallisierte.

Das erhaltene Rohprodukt wurde in 1,93 l Aceton gelöst und mit 65 ml konzentrierter Salzsäure versetzt. Nach beginnender Kristallisation wurde eine Stunde unter Eisbadkühlung gerührt, bevor der Niederschlag abgesaugt wurde. Es wurde zweimal mit Aceton und mit Diethylether gewaschen und anschließend das Kristallisat im Ölpumpenvakuum bei 70° C bis zur Gewichtskonstanz getrocknet. Es wurden 216,8 g (96 % der Theorie) farblose Kristalle erhalten.

Schmelzpunkt: 247-248° C (Zersetzung)
Drehwert: $[\alpha]_D^{RT}$ = -35,2° (c = 1,00; Methanol)

**Beispiel 2**

(+)-(1R, 2R)-2-Dimethylaminomethyl-1-(3-methoxy-phenyl)-cyclohexanol, Hydrochlorid (+1)

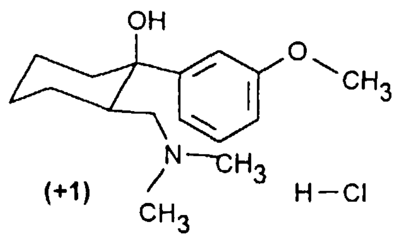

(+1)

1. Stufe: Freisetzen der Base aus der Mutterlauge der L-(+)-Weinsäure-Fällung

Die ethanolische Mutterlauge sowie die Waschphase aus der L-(+)-Weinsäurefällung (Beispiel 1, 2. Stufe) wurden vereinigt. Nach destillativer Entfernung des Lösungsmittels wurde der Rückstand (2080 g) in 2500 ml Wasser gelöst und mit 900 g zerstoßenem Eis versetzt. Unter Rühren wurden 1000 ml 36-38 %ige Natronlauge (technisch) zugetropft. Anschließend wurde mit 2700 ml Dichlormethan und nach Phasentrennung mit weiteren 600 ml Dichlormethan extrahiert. Die vereinigten organischen Phasen wurden über Natriumsulfat getrocknet. Nach destillativer Entfernung des Lösungsmittels wurden 1340 g (99 % der Theorie) (1R, 2R)-2-Dimethylaminomethyl-1-(3-methoxy-phenyl)-cyclohexanol als Sirup erhalten.

2. Stufe: Überführung von (1R, 2R)-2-Dimethylamino-methyl-1-(3-methoxy-phenyl)-cyclohexanol in das Hydrochlorid (+1)

1340 g (5,09 mol) der aus Stufe 1 erhaltenen Base wurden in 17,5 l 2-Butanon gelöst und unter Rühren mit 105 ml (5,8 mol) Wasser sowie 670 ml (573 g; 5,3 mol) Trimethylchlorsilan versetzt. Zur Kristallisation wurde 3 Stunden bei Raumtemperatur gerührt und 24 Stunden bei dieser Temperatur stehen gelassen. Der ausgefallene Feststoff wurde abgesaugt, mit 5000 ml 2-Butanon gewaschen und bei 90° C im Vakuum (60 mbar) bis zur Gewichtskonstanz getrocknet. Es wurden 1350 g (88 % der Theorie bezogen auf die eingesetzte Base aus Stufe 1 und 89 % bezogen auf den Enantiomerenanteil des eingesetzten Racemates aus Beispiel 1, Stufe 1) Hydrochlorid (+1) als farblose Kristalle erhalten.

| | |
|---|---|
| Schmelzpunkt: | 171-172° C |
| Drehwert: | $[\alpha]_D^{RT}$ = +29,6° (c = 1,00; Methanol) |

3. Stufe: Überführung von Hydrochlorid (+1) in (+)-(1R, 2R)-3-(2-Dimethylaminomethyl-1-hydroxy-cyclohexyl)-phenol, Hydrochlorid

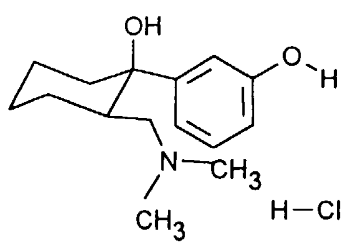

Ausgehend von Hydrochlorid (+1) wurde unter den in Beispiel 1, 5. Stufe angegebenen Bedingungen das (+)-Enantiomere von O-Demethyltramadol als Hydrochlorid in 96 %iger Ausbeute erhalten.

| | |
|---|---|
| Schmelzpunkt: | 247-248° C (Zersetzung) |
| Drehwert: | $[\alpha]_D^{RT}$ = +35,4° (c = 1,00; Methanol) |

**Pharmakologische Untersuchungen**

**Analgesieprüfung im Writhing-Test an der Maus**

Die Untersuchung auf analgetische Wirksamkeit wurde im Phenylchinon-induzierten Writhing an der Maus (modifiziert nach I.C. Hendershot, J. Forsaith, J. Pharmacol. Exp. Ther. 125, 237-240 (1959)) durchgeführt. Dazu wurden männliche NMRI-Mäuse mit einem Gewicht von 25-30 g verwendet. Gruppen von 10 Tieren pro Substanzdosis

erhielten 10 Minuten nach intravenöser Gabe der erfindungsgemäß zu verwendenden Verbindungen 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45° C) intraperitoneal appliziert. Die Tiere wurden einzeln in Beobachtungskäfige gesetzt und mittels Drucktastenzähler die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Aus der dosisabhängigen Abnahme der Writhingreaktionen im Vergleich zu parallel untersuchten nur mit Phenylchinon behandelten Kontrollgruppen wurden mittels Regressionsanalyse (Auswerteprogramm Martens EDV Service, Eckental) die $ED_{50}$-Werte mit 95 % Vertrauensbereich der Writhingreaktion berechnet.

**Analgesieprüfung im Tailflick-Test an der Ratte**

Die analgetische Wirksamkeit der erfindungsgemäß zu verwendenden Verbindungen wurde im Wärmestrahl (Tailflick)-Test an der Ratte nach der Methode von D'Amour und Smith (J. Pharm. Exp. Ther. 72, 74-79 (1941) untersucht. Dazu wurden weibliche Sprague Dawley Ratten mit einem Gewicht von 120 - 160 g verwendet. Die Tiere wurden einzeln in Testkäfige gesetzt und die Schwanzbasis einem focussierten Wärmestrahl einer elektrischen Lampe (Rhema Analgesiemeter für Ratten) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 3 - 6 Sekunden betrug. Vor Gabe der erfindungsgemäß zu verwendenden Verbindungen wurden die Tiere innerhalb von fünf Minuten zweimal getestet und der Mittelwert dieser Messungen als Vortestmittelwert berechnet. Die Schmerzmessung wurde 20, 40 und 60 Minuten nach intravenöser Gabe durchgeführt. Bei Anstieg der Schmerzlatenz wurde die maximale Expositionszeit auf 12 Sekunden beschränkt und die Zunahme der Latenzzeit auf > 150 % des Vortestmittelwertes als analgetische Wirkung gewertet. Zur Bestimmung der Dosisabhängigkeit wurden die Verbindungen in 3 - 5 logarithmisch ansteigenden Dosen, die jeweils die Schwellen- und die maximale Wirkdosis einschlossen, appliziert und aus der Anzahl der analgetischen Tiere nach der Methode von Litchfield und Wilcoxon (J. Pharm. Exp. Ther. 96, 99-113 (1949)) die $ED_{50}$-Werte bestimmt. Die $ED_{50}$-Berechnung erfolgte im Wirkmaximum 20 Minuten nach intravenöser Substanzgabe.

Die erfindungsgemäß zu verwendenden Enantiomeren von O-Demethyltramadol zeigten im Writhing-Test an der Maus und im Tailflick-Test an der Ratte eine ausgeprägte analgetische Wirkung. Die Ergebnisse sind in der nachfolgenden Tabelle zusammengestellt.

Tabelle:

| Analgesieprüfung im Writhing-Test an der Maus und Tailflick-Test an der Ratte | | |
|---|---|---|
| Verbindung | $ED_{50}$ (mg/kg, intravenös) im Writhing-Test | $ED_{50}$ (mg/kg, intravenös) im Tailflick-Test |
| (+)-Enantiomer von O-Demethyltramadol, Hydrochlorid | 0,489 | 1,01 |
| (-)-Enantiomer von O-Demethyltramadol, Hydrochlorid | 6,60 | >10,0 |
| zum Vergleich: racemisches Tramadolhydrochlorid | 3,59 | 6,47 |

**Patentansprüche**

1. Verfahren zur Herstellung der Enantiomeren von O-Demethyltramadol, dadurch gekennzeichnet, daß man ein racemisches Tramadolsalz in die Base überführt, mit L-(+)-Weinsäure das (-)-Tramadol-Enantiomere durch Fällung abtrennt und nach Freisetzung der Tramadolbase in das (-)Enantiomere von O-Demethyltramadol mit Diisobutylaluminiumhydrid überführt, und aus der Mutterlauge der Weinsäurefällung das (+)-Enantiomere von O-Demethyltramadol durch Freisetzung der Tramadolbase und Umsetzung mit Diisobutylaluminiumhydrid herstellt.

2. Verfahren nach Anspruch 1, dadurch gekennzeichnet, daß racemisches Tramadolhydrochlorid eingesetzt wird.

3. Verfahren nach einem oder beiden der Ansprüche 1 bis 2, dadurch gekennzeichnet, daß L-(+)-Weinsäure in Gegenwart eines organischen Lösungsmittels, vorzugsweise in Gegenwart eines aliphatischen $C_{1-5}$-Alkohols eingesetzt wird.

**4.** Verfahren nach einem oder mehreren der Ansprüche 1 bis 3, dadurch gekennzeichnet, daß das (-)-Tramadol-Enantiomere durch Kristallisation abgetrennt wird.

**5.** Verfahren nach einem oder mehreren der Ansprüche 1 bis 4, dadurch gekennzeichnet, daß man vor der Umsetzung mit Diisobutylaluminiumhydrid das entsprechende Enantiomere der Tramadolbase in ein von Tartrat verschiedenes Salz überführt, aus welchem anschließend die Base freigesetzt wird.

**6.** Verfahren nach Anspruch 5, dadurch gekennzeichnet, daß das entsprechende Enantiomere der Tramadolbase in das Hydrochlorid überführt wird.

**Claims**

**1.** Process for preparing the enantiomers of O-demethyltramadol, characterised in that a racemic tramadol salt is converted into the base, the (-)-tramadol enantiomer is separated off by precipitation with L-(+)-tartaric acid and, after release of the tramadol base, is converted by means of diisobutylaluminium hydride into the (-)-enantiomer of O-demethyltramadol and, from the mother liquor of the tartaric acid precipitation, the (+)-enantiomer of O-demethyltramadol is prepared by release of the tramadol base and reaction with diisobutylaluminium hydride.

**2.** Process according to claim 1, characterised in that racemic tramadol hydrochloride is used.

**3.** Process according to one or both of claims 1 and 2, characterised in that L-(+)-tartaric acid is used in the presence of an organic solvent, preferably in the presence of an aliphatic $C_{1-5}$ alcohol.

**4.** Process according to one or more of claims 1 to 3, characterised in that the (-)-tramadol enantiomer is separated off by crystallisation.

**5.** Process according to one or more of claims 1 to 4, characterised in that, prior to the reaction with diisobutylaluminium hydride, the corresponding enantiomer of the tramadol base is converted into a salt other than tartrate, from which other salt the base is then released.

**6.** Process according to claim 5, characterised in that the corresponding enantiomer of the tramadol base is converted into the hydrochloride.

**Revendications**

**1.** Procédé de préparation des énantiomères du O-déméthyltramadol, caractérisé en ce qu'on transforme un sel de tramadol racémique en la base, on sépare le (-)-énantiomère de tramadol par précipitation avec l'acide L-(+)-tartrique et après libération du tramadol-base, on le transforme avec l'hydrure de diisobutylaluminium en le (-)-énantiomère du O-déméthyltramadol, et à partir de la liqueur-mère de précipitation par l'acide tartrique, on prépare le (+)-énantiomère du O-déméthyltramadol par libération du tramadol-base et réaction avec l'hydrure de diisobutylaluminium.

**2.** Procédé suivant la revendication 1, caractérisé en ce qu'on utilise du chlorhydrate de tramadol racémique.

**3.** Procédé suivant l'une des revendications 1 et 2 ou les deux, caractérisé en ce qu'on utilise l'acide L-(+)-tartrique en présence d'un solvant organique, de préférence en présence d'un alcool aliphatique en $C_1$ à $C_5$.

**4.** Procédé suivant une ou plusieurs des revendications 1 à 3, caractérisé en ce que le (-)-énantiomère de tramadol est séparé par cristallisation.

**5.** Procédé suivant une ou plusieurs des revendications 1 à 4, caractérisé en ce que, préalablement à la réaction avec l'hydrure de diisobutylaluminium, on transforme l'énantiomère correspondant du tramadol-base en un sel différent du tartrate, duquel on libère ensuite la base.

**6.** Procédé suivant la revendication 5, caractérisé en ce que l'énantiomère correspondant du tramadol-base est transformé en le chlorhydrate.